# EUROPEAN PATENT SPECIFICATION

(11) **EP 3 354 252 B1**
(45) Date of publication and mention of the grant of the patent: **04.05.2022**
(21) Application number: 16839530.9
(22) Date of filing: 19.08.2016
(51) Int. Cl.: A61K 8/19, A61Q 5/00, A61Q 5/04

(54) **HAIR PERM FORMING OXIDIZING AGENT CONTAINING HYDROGEN WATER, AND HAIR PERM FORMING METHOD USING SAME**
OXIDATIONSMITTEL FÜR DAUERWELLEN MIT WASSERSTOFFWASSER UND VERFAHREN ZUM FORMEN EINER DAUERWELLE DAMIT
AGENT OXYDANT DESTINÉ À PERMANENTE DES CHEVEUX CONTENANT DE L'EAU HYDROGÉNÉE, ET MÉTHODE DE PERMANENTE DES CHEVEUX CORRESPONDANTE

(30) Priority: 21.08.2015 KR 20150118239
(43) Date of publication of application: 01.08.2018
(73) Proprietor: Konkuk University Industrial Cooperation Corp., Gwangjin-gu Seoul 05029 (KR); Jeon, Byoung-moon, Seoul 06134 (KR)
(72) Inventor: JEON, Byoung-moon, Seoul 06134 (KR); KANS, Sangmo, Seoul 04426 (KR)
(74) Representative: EP&C
(86) International application number: PCT/KR2016/009195
(87) International publication number: WO 2017/034241

(56) References cited:
- JP-A- 2004 018 509
- JP-A- 2005 187 447
- JP-A- 2010 100 559
- JP-B2- 5 690 881
- JP-B2- 5 734 898
- DATABASE WPI Week 201512 Thomson Scientific, London, GB; AN 2015-09650F XP002792082, & JP 2015 017064 A (KURIMURA M) 29 January 2015 (2015-01-29)
- DATABASE WPI Week 201529 Thomson Scientific, London, GB; AN 2015-24600B XP002792083, & WO 2015/053225 A1 (NAGAOKA Y) 16 April 2015 (2015-04-16)
- DATABASE GNPD [Online] MINTEL; 1 July 2013 (2013-07-01), "Revitalize Booster Lotion", XP002792084, Database accession no. 2132633
- DATABASE GNPD [Online] MINTEL; 2 March 2015 (2015-03-02), "Micro Water", XP002792085, Database accession no. 2984705
- DATABASE WPI Week 200552 Thomson Scientific, London, GB; AN 2005-508747 XP002792086, & JP 2005 187447 A (INAGAKI T) 14 July 2005 (2005-07-14)

## Description

### Technical Field

The present embodiment relates to an oxidizing agent for forming a hair perm, including hydrogen water, and to a method of forming a hair perm using the same.

### Background Art

The following description merely provides background information related to the present embodiment, and does not constitute the related art.

With the development of scientific technology and medicine, the desire to extend the life expectancy of humans gradually increases, and thus the pursuit of a relaxed life and the desire to maintain beautiful skin are growing. Interest in environmental friendliness, naturalism, health and the like in modern society have become important in the beauty and fashion fields that are directly applied to the human body. However, in the beauty field, studies on permanent-waving procedures, which frequently use chemical products, are mostly related to reducing agents, and research into oxidizing agents that reform the tertiary structure of keratin is lacking.

A perm agent for a two-bath process conventionally used for a hair perm includes a reducing agent that breaks some of the disulfide bonds of the hair and an oxidizing agent that recombines some thereof. In the reducing agent and the oxidizing agent, examples of the oxidizing agent include hydrogen peroxide (HO) and sodium bromate (Br). In many hair-perm-related studies, permed hair using an oxidizing agent for a hair perm is troubled by dryness, resistance, and color changes. In particular, the International Agency for Research on Cancer (IARC) classifies bromate as a potentially carcinogenic "Group 2B" substance. Bromate is easy to use in cosmetic agents, but may cause renal failure, hearing loss, etc. upon poisoning thereof, and may result in death, and is thus regarded as a dangerous chemical. An existing oxidizing agent has a polar pH different from that of the reducing agent, thus making the hair and scalp weakly resistant to sudden changes, causing hair bleaching, damage to the hair, and trouble to the scalp, weakening resistance and incurring DNA denaturation. Moreover, in accordance with the National Institute of Food and Drug Safety Evaluation, Korea, hydrogen peroxide (HO), which is an existing oxidizing agent, is problematic because stinging pain and whitening may occur upon contact at low concentration with the skin and also because severe ulcerative burns, flares, stinging pain, tearing and blurred vision may occur upon contact at high concentration with the skin.

With the goal of solving the problems with existing oxidizing agents, research is ongoing into the use, as an oxidizing agent for a hair perm, of basic electrolytic water obtained by electrolysis of water, which is a natural resource. In Japan, the development of an oxidizing agent for a hair perm using acidic electrolytic water has been conducted. Therefore, thorough research and continuous development is required for the health of human bodies and the environment.

In JP2015017064 a curly hair-correcting method is provided as well as an auxiliary agent for curly hair correction which can achieve suitable curly hair correction, deterioration suppression of a feeling of the hair, or damage suppression to the hair. It uses hydrogen peroxide or sodium bromate as oxidizing agent. Hydrogen water is not used as oxidizing agent. In JP2005187447 a hair-dying composition is provided which is constituted by henna as a first agent and hydrogen water as a second agent. It does not relate to a method of forming a hair perm. In WO2015/053225 treatment agent is provided which is capable of effectively preventing oxidative stress caused by reactive oxygen species or free radical in skin or hair. Hydrogen water is used to prevent oxidative stress on skin or hair, and is not used as an oxidizing agent in perm.

### Disclosure

### Technical Problem

Accordingly, the present embodiment is intended to provide a method of forming a hair perm using an oxidizing agent, in which side effects of existing oxidizing agents for forming a hair perm, such as hydrogen peroxide (HO) and sodium bromate (Br), on the scalp and hair may be alleviated, and excellent curl formability and sustainability may be maintained.

### Technical Solution

In accordance with an aspect of the present embodiment, a method of forming a hair perm using hydrogen water as the unique oxidizing agent is provided, wherein the hydrogen water has a pH of 7.0 to 7.9. The hydrogen water may have a dissolved hydrogen content of at least 100 ppb.

### Advantageous Effects

According to the present embodiment, an oxidizing agent for forming a hair perm is capable of alleviating side effects of existing oxidizing agents for forming a hair perm, such as hydrogen peroxide (HO) and sodium bromate (Br), on the scalp and hair, is harmless to human bodies, and is effective at maintaining excellent curl formability and sustainability.

According to the present embodiment, a method of forming a hair perm using an oxidizing agent can be provided, the oxidizing agent being capable of alleviating side effects of existing oxidizing agents for forming a hair perm, such as hydrogen peroxide (HO) and sodium bromate (Br), on the scalp and hair, and exhibiting excellent curl formability and sustainability.

Generally, in the case of bleached hair, the hair epidermis and hair cortex, which form hair curls, are damaged during the bleaching process. Even if hair perming is further carried out on the bleached hair, it is not easy to recombine the structure of the hair, making it difficult to exhibit a hair-perming effect. However, in the test of the present embodiment, hydrogen water is used in lieu of hydrogen peroxide (HO) and sodium bromate (Br), which are existing oxidizing agents, during the process of perming the bleached hair, thereby effectively maintaining the curl formability and sustainability.

### Description of Drawings

FIG. 1 shows a normal perm procedure in the process of forming a hair perm according to the present embodiment;
FIG. 2 shows a heat perm procedure in the process of forming a hair perm according to the present embodiment;
FIG. 3 is a photograph showing the hair samples used for the test of the present embodiment;
FIG. 4 is a photograph showing the hair group using sodium bromate (Br) as an oxidizing agent and the hair group using hydrogen water (HW) having a dissolved hydrogen content of 100 ppb as an oxidizing agent according to the present embodiment;
FIG. 5 is a photograph showing the hair group using hydrogen peroxide (HO) as an oxidizing agent and the hair group using, as an oxidizing agent, hydrogen water (HW) obtained after the operation of a hydrogen water generator for about 30 min according to the present embodiment;
FIG. 6 is a photograph showing the hair group using hydrogen peroxide (HO) as an oxidizing agent, the hair group using, as an oxidizing agent, hydrogen water (HW) obtained after the operation of a hydrogen water generator for about 30 min, and the hair group using, as an oxidizing agent, hydrogen water (HW) obtained after the operation of a hydrogen water generator for about 150 min according to the present embodiment; and
FIG. 7 is of photographs showing the results of use of hydrogen water having dissolved hydrogen, obtained after the operation of a hydrogen water generator for about 150 min, as the oxidizing agent during a heat perm procedure by direct application to a model according to the present embodiment.

### Mode for Invention

The present inventors have made efforts to alleviate side effects of existing oxidizing agents for forming a hair perm, such as hydrogen peroxide (HO) and sodium bromate (Br), on the scalp and hair, and have ascertained that when hydrogen water (HW), which is harmless to human bodies and has not been used to date to form hair perms, is used as an oxidizing agent for forming a hair perm, very good effects may be exhibited, thus culminating in the present invention.

Hereinafter, a detailed description will be given of the present embodiment with reference to the appended drawings.

According to the present embodiment, an oxidizing agent for forming a hair perm includes hydrogen water (HW). The hydrogen water (HW) may be simply and easily prepared using a commercially available hydrogen water generator. In the present embodiment, a product made by Lourdes is used as the hydrogen water generator. The concentration of dissolved hydrogen may be easily controlled by adjusting the operation time of the hydrogen water generator. The hydrogen water (HW) preferably has a dissolved hydrogen content of at least 100 ppb. When the dissolved hydrogen content of the hydrogen water (HW) is at least 100 ppb, the hydrogen water is harmless to human bodies and curl formability and sustainability may be exhibited at a level similar to those of existing oxidizing agents such as hydrogen peroxide (HO) and sodium bromate (Br).

In the present embodiment, as the concentration of dissolved hydrogen in hydrogen water (HW) is increased, higher curl formability and sustainability may be exhibited (100 ppb < 400 ppb < 1,000 ppb < 2,000 ppb < 2,500 ppb < 4,400 ppb). The concentration of dissolved hydrogen in hydrogen water (HW) is preferably at least 400 ppb. When the concentration of dissolved hydrogen in hydrogen water (HW) is at least 400 ppb, such hydrogen water is harmless to human bodies and curl formability and sustainability superior to those of existing oxidizing agents, such as hydrogen peroxide (HO) and sodium bromate (Br), may be exhibited. When the concentration of dissolved hydrogen in hydrogen water (HW) is at least about 1,000 ppb, the hydrogen water is harmless to human bodies, and curl formability and sustainability remarkably superior to those of existing oxidizing agents, such as hydrogen peroxide (HO) and sodium bromate (Br), may be exhibited. The concentration range of dissolved hydrogen in hydrogen water may vary depending in part on the hydrogen water generator, but all concentration values fall within the scope of the present invention.

According to the present embodiment, the oxidizing agent for forming a hair perm including hydrogen water (HW) has a pH of about 7 to 7.3, and may thus be stably applied to the hair and scalp. The pH range of the hydrogen water (HW) may vary depending in part on the hydrogen water generator, but all pH values fall within the scope of the present invention. The oxidizing agent having a pH of about 7 to 7.9 may be stably applied to the hair and scalp.

The present embodiment addresses a method of forming a hair perm using hydrogen water as the unique oxidizing agent.

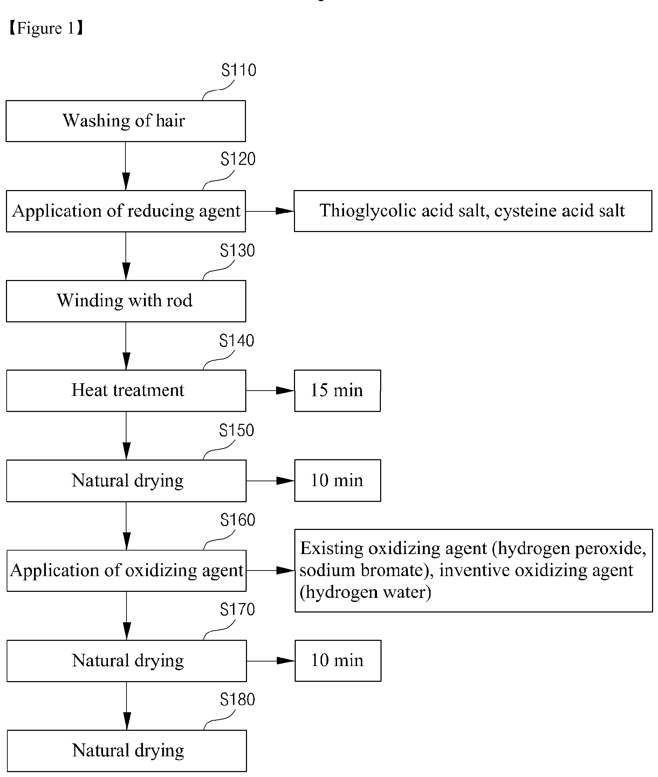

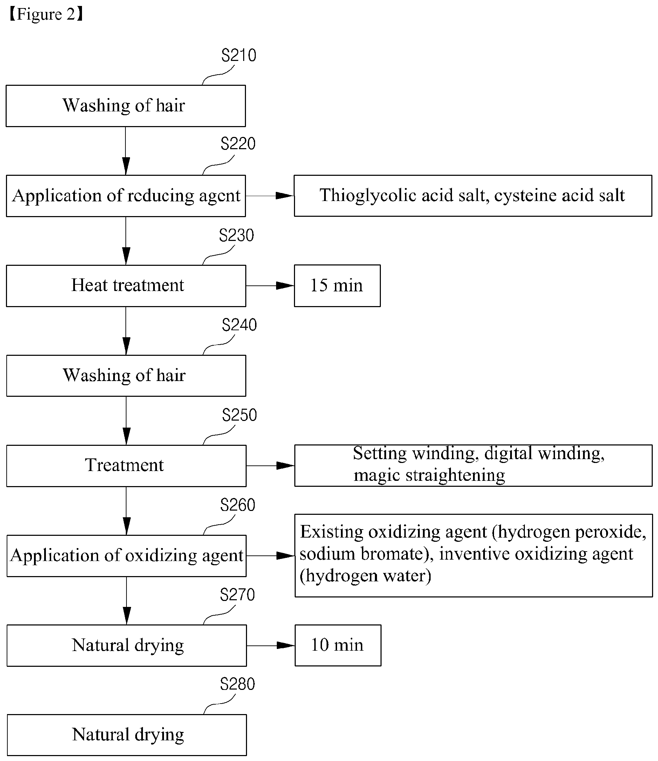

The method of forming a hair perm may be applied to both the normal perm process and the heat perm process of FIGS. 1 and 2. The method of forming a hair perm obviates the use of existing oxidizing agents such as hydrogen peroxide (HO) and sodium bromate (Br) and is thus harmless to human bodies, and may exhibit high curl formability and sustainability.

FIG. 1 shows the normal perm process. The hair is washed (S110), after which a reducing agent (thioglycolic acid salt, cysteine acid salt) is applied on the hair (S120) to cleave cystine bonding of the hair, thus unwinding the typical shape of the hair. The hair having the reducing agent applied thereon is fixed to a desired shape using rods (S130). Thereafter, a chemical reaction of the hair (on which the reducing agent is applied) wound with the rods is rapidly carried out using heat for a preset period of time (e.g. 15 min) (S140). Here, a heat treatment step (S140) may be omitted. Thereafter, the hair is subjected to natural drying as non-heat treatment for a preset period of time (e.g. 10 min) (S150). An oxidizing agent is applied on the hair to induce cystine bonding of the hair (S160). In the present embodiment, during S160 of FIG. 1, hydrogen water (HW) is used in lieu of existing oxidizing agents (hydrogen peroxide (HO), sodium bromate (Br)), thus completing the shape of the hair. In the course of inducing the cystine bonding of the hair by the function of the oxidizing agent by applying hydrogen water (HW) on the hair, the shape of the hair may be completed due to the presence of hydrogen radicals. The hydrogen water (HW) according to the present embodiment may have a dissolved hydrogen content of about 100 ppb to 4,400 ppb. The hair having the oxidizing agent (hydrogen water) applied thereon is naturally dried for a preset period of time (e.g. 10 min) (S170) and then washed (S180).

FIG. 2 shows the heat perm process. The hair is washed (S210), after which a reducing agent (thioglycolic acid salt, cysteine acid salt) is applied on the hair (S220) to cleave cystine bonding of the hair, thus unwinding the typical shape of the hair. Thereafter, a chemical reaction of the hair having the reducing agent applied thereon is rapidly carried out using heat for a preset period of time (e.g. 15 min) (S230). Here, a heat treatment step (S230) may be omitted. The hair is washed (S240) so as to prevent the chemical reaction of the reducing agent from directly occurring by means of heat applied after the reducing agent application step (S220) or the heat treatment step (S230). The shape of the hair is fixed using a heat perm device (a hair perm setting device, a digital perm device, a magic straightening device) (S250). An oxidizing agent is applied on the hair to induce cystine bonding of the hair (S260). In the present embodiment, during S260 of FIG. 2, hydrogen water (HW) is used in lieu of existing oxidizing agents (hydrogen peroxide (HO), sodium bromate (Br)), thus completing the shape of the hair. In the course of inducing the cystine bonding of the hair by the function of the oxidizing agent by applying hydrogen water (HW) on the hair, the shape of the hair may be completed due to the presence of hydrogen radicals. The hydrogen water (HW) according to the present embodiment may have a dissolved hydrogen content of about 100 ppb to 4,400 ppb. The hair having the oxidizing agent (hydrogen water) applied thereon is naturally dried for a preset period of time (e.g. 10 min) (S270) and then washed (S280).

A better understanding of the present embodiment will be given through the following preferred Examples, which are merely set forth to illustrate but are not to be construed as limiting the scope of the present embodiment.

In the present embodiment, a test sample was made using the hair of a female model in her twenties (resident of Seoul), and testing was conducted in the same manner as in the hair perm process in a hair salon. As test results, the curl formability and sustainability of the hair were confirmed and the extent of damage to the hair and scalp was observed by a beauty expert (having about 20 years of experience) .

### [Examples]

Hydrogen water (HW) was obtained using a conventionally commercially available hydrogen water generator. In the present embodiment, water having a dissolved hydrogen content of 100 ppb or more was stepwisely produced up to a maximum of 4,400 ppb using a hydrogen water generator. The numerical value of dissolved hydrogen content is increased depending on the amount of water and the saturation concentration thereof. Specifically, the numerical value of dissolved hydrogen content may be accurately measured only up to 2,000 ppb due to the limitation of the measurement range of the measuring device. However, in practice, as the value of ppb is higher, curl formability may increase during the hair perm process, and the value of ppb may be set to at least about 4,400 ppb. The hydrogen water (HW) obtained using the provided hydrogen water generator has a pH of 7.0 to 7.3.

The pH range of hydrogen water (HW) may vary depending in part on the hydrogen water generator. Specifically, the pH of the oxidizing agent for forming a hair perm, including hydrogen water (HW), is preferably about 7 to 7.3, but is not limited thereto. The oxidizing agent having a pH of 7 to 7.9 may be stably applied to the hair and scalp.

The hydrogen water (HW) applied to the hair sample is hydrogen water (HW) having a dissolved hydrogen content ranging from 100 ppb to 4,400 ppb, and a total of 400 g thereof was divided in two, and 200 g of the hydrogen water was applied two times to the hair. 200 g of HW was first applied on the hair, and after 10 min, 200 g of HW was further applied on the hair, and the perm process was performed in the same manner as in a typical perm process in a hair salon. The hair sample used during the perm process using the hydrogen water (HW) as the oxidizing agent was made by directly cutting the hair of the same female model to the same length and weight (3 g, 20 cm long).

In the test to confirm the likelihood of completing a hair perm using hydrogen water (HW) as the oxidizing agent, as a control group, existing oxidizing agents such as sodium bromate (Br) and hydrogen peroxide (HO) were used, and as a test group, a hydrogen water (HW) oxidizing agent was used.

The hair sample having a length of 20 cm obtained from the female model was washed and a commercial perm agent made by ADC, as a reducing agent (a first perm agent), was applied on the hair sample. After the application of the reducing agent, winding, heat treatment and natural drying steps were performed, and as oxidizing agents, sodium bromate (Br), hydrogen peroxide (HO), and hydrogen water (HW) were used.

In order to measure the curl formability, hair having the same length and weight as above was treated in the same manner as in the perm process in the hair salon, and was then wound by the same experimenter using rods having the same thickness.

In order to compare the perm results, the extent of decreasing the total hair length before and after testing was measured. Specifically, the hair sample was hung on the grid paper, and a decrease in the total length of the curled hair in spring form due to the perm process was compared before and after testing. The extent to which the total length of the hair was decreased was determined to be the curl formability.

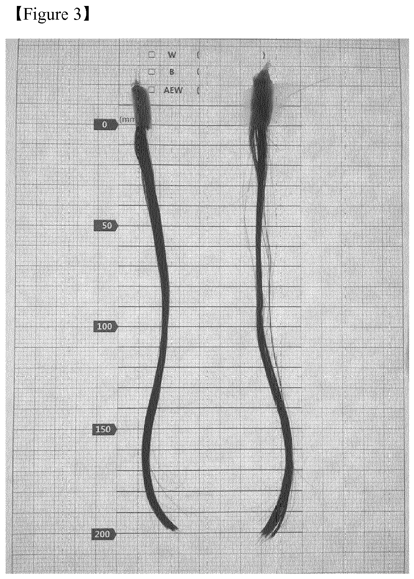

FIG. 3 is a photograph in which the length of the hair sample is measured before the test. The hair sample was made by directly cutting the hair of the female resident of Seoul in her twenties to the same length and weight (3 g, 20 cm long), and the upper hair portion in the direction of the hair follicle was fixed by silicone and taping so as not to come loose. In order to eliminate the perception of the experimenter on the sample, pretreatment and winding were performed under the same conditions without marking before the oxidizing agent treatment step during the hair perm process.

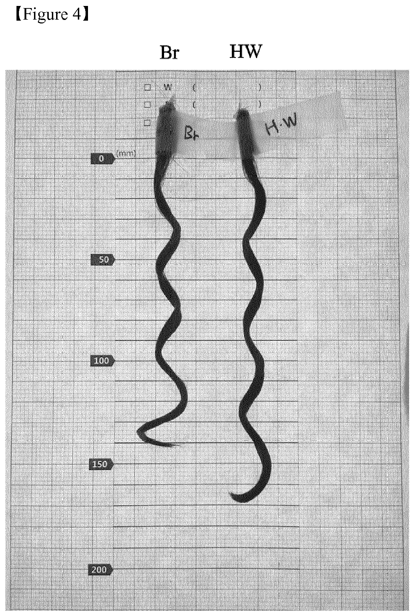

FIG. 4 is a photograph showing the hair group using sodium bromate (Br) as an oxidizing agent and the hair group using hydrogen water (HW) having a dissolved hydrogen content of 100 ppb as an oxidizing agent according to the present embodiment. Specifically, FIG. 4 is a photograph showing the test results using hydrogen water (HW) having a dissolved hydrogen content of 100 ppb (operation of a hydrogen water generator for about 5 min) as an oxidizing agent for a hair perm. The control group was sodium bromate (Br), and when using sodium bromate (Br), the length of the hair was decreased by about 6 cm, from 20 cm before the hair perm process to about 14 cm after the hair perm process, as shown in FIG. 3. When hydrogen water (HW) was used as the oxidizing agent for a hair perm, the length of the hair was decreased by about 3 cm, from 20 cm before the hair perm process to about 17 cm. Based on the test results, curls were formed in both of the two hair samples.

<Table 1> below shows the results of measurement of curl sustainability of the hair, as shown in FIG. 4. Also based on the results of measurement of curl sustainability, the hair was shampooed every day for 14 days after the perm process, after which the length of the hair was similarly increased by 9 mm in the case of sodium bromate (Br) and by 11 mm in the case of hydrogen water (HW) .

**[Table 1]**

| Unit (mm)/(200 mm length difference) | Sodium bromate (Br) | Hydrogen water (HW) |
|---|---|---|
| Before treatment | 200 | 200 |
| After treatment | 142 (58) | 169 (31) |
| After 7 days | 147 (53) | 176 (24) |
| After 14 days | 151 (49) | 180 (20) |

As is apparent from <Table 1>, based on the curl sustainability results in the hair perm process using hydrogen water (HW) as the oxidizing agent, the length of curled hair was increased by a similar ratio for sodium bromate (Br) and hydrogen water (HW). Since the increase in the length of the curled hair was not large over time, the shape of the curl when using hydrogen water as the oxidizing agent at a hair salon was sustained, like existing oxidizing agents.

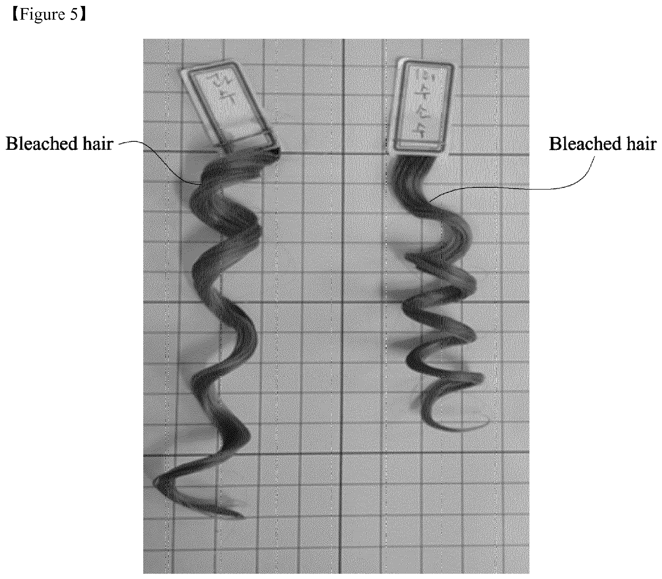

FIG. 5 is a photograph showing the hair group using hydrogen peroxide (HO) as an oxidizing agent and the hair group using, as an oxidizing agent, hydrogen water (HW) obtained after operation of a hydrogen water generator for about 30 min according to the present embodiment. Specifically, FIG. 5 is a photograph showing the test results of bleached hair when hydrogen water (HW) having a dissolved water content of 1,000 ppb (operation of a hydrogen water generator for about 30 min) was used as the oxidizing agent for a hair perm. The curling of the hair was better when using hydrogen water (HW) than when using hydrogen peroxide (HO) as the oxidizing agent.

Therefore, it was confirmed that hydrogen water (HW) having a dissolved hydrogen content of at least 1,000 ppb could be used as the oxidizing agent for a hair perm in the hair salon, in lieu of hydrogen peroxide (HO), for the bleached hair.

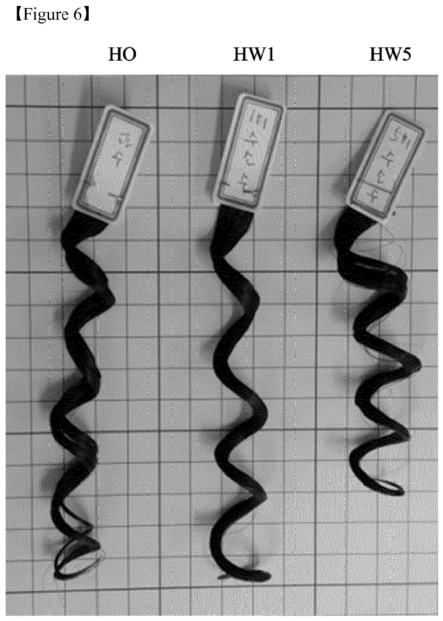

FIG. 6 is a photograph showing the hair group using hydrogen peroxide (HO) as an oxidizing agent, the hair group using hydrogen water (HW) obtained after operation of a hydrogen water generator for about 30 min, and the hair group using hydrogen water (HW) obtained after operation of a hydrogen water generator for about 150 min according to the present embodiment. Specifically, FIG. 6 is a photograph showing the test results of human hair when using hydrogen water (HW) having a dissolved hydrogen content of about 4,400 ppb (operation of a hydrogen water generator for about 150 min) as the oxidizing agent for a hair perm. When comparing the hair control group using hydrogen peroxide (HO) as the oxidizing agent with the hair group (HW1) using hydrogen water (HW) having a dissolved hydrogen content of about 1,000 ppb (operation of a hydrogen water generator for about 30 min) as the oxidizing agent and the hair group (HW5) using hydrogen water (HW) having a dissolved hydrogen content of about 4,400 ppb (operation of hydrogen water generator for about 150 min) as the oxidizing agent, the curling of the hair was better in the hair group (HW5) using hydrogen water (HW) having a dissolved hydrogen content of about 4,400 ppb than in the hair group using existing hydrogen peroxide (HO) as the oxidizing agent.

<Table 2> below shows, as the test results of FIGS. 5 and 6, the numerical values of the curl formability by measuring the extent of decrease of the hair length before and after the hair perm process. The curl formability for bleached hair was superior by 13% in Sample B, using hydrogen water having a dissolved hydrogen content of about 1,000 ppb (operation of a hydrogen water generator for about 30 min) as the oxidizing agent, compared to Sample A, using hydrogen peroxide (HO) (FIG. 5).

**[Table 2]**

| | Sample | Length (cm) | Curl formability (Ratio before and after treatment) |
|---|---|---|---|
| A | Bleached hair (Hydrogen peroxide) | 13 | 56.5% |
| B | Bleached hair Dissolved hydrogen content of 1,000 ppb (operation of hydrogen water generator for about 30 min) | 13 | 43.5% |
| C | Normal hair (Hydrogen peroxide) | 12.5 | 54.3% |
| D | Normal hair Dissolved hydrogen content of 1,000 ppb (operation of hydrogen water generator for about 30 min) | 13 | 56.5% |
| E | Normal hair Dissolved hydrogen content of 4,400 ppb (operation of hydrogen water generator for about 150 min) | 10 | 43.5% |

As is apparent from <Table 2>, the curl formability for normal hair was superior by 10.8% in Sample E, using hydrogen water (HW) having a dissolved hydrogen content of about 4,400 ppb (operation of a hydrogen water generator for about 150 min) as the oxidizing agent, compared to Sample C, using hydrogen peroxide (HO) as the oxidizing agent. Based on the aforementioned results, in the case of bleached hair and normal hair, the curling was better when using hydrogen water (HW) as the oxidizing agent than when using hydrogen peroxide (HO) and sodium bromate (Br) as the oxidizing agents.

In normal human hair, no substances that exhibit high curl formability compared to when using hydrogen peroxide (HO) or sodium bromate (Br) have yet been found, and high-concentration hydrogen water showed outstanding curl formability in normal human hair compared to existing oxidizing agents. The efficacy of hydrogen water applied to human hair is deemed to be an effect that has not been discovered to date. For reference, normal human hair is hair that is not subjected to any treatment, such as perming, dyeing or bleaching, and thus does not curl as well as damaged hair. Furthermore, Sample D, using hydrogen water (HW) having a dissolved hydrogen content of about 1,000 ppb as the oxidizing agent, exhibited curl formability different by only 0.5 cm from that of Sample C, such a difference being imperceptible even to hair experts. The bleached hair exhibits low curl formability and is not subjected to the hair perm process, but hydrogen water (HW) may be favorably applied thereto.

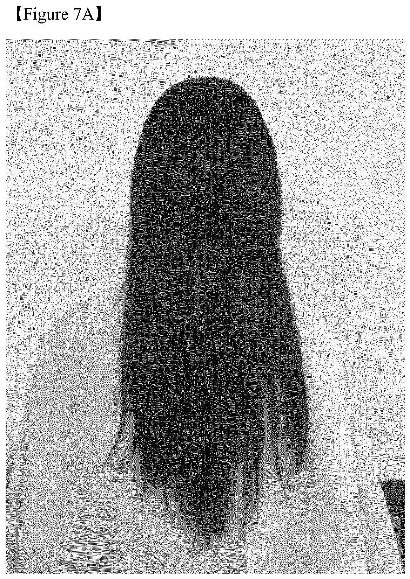

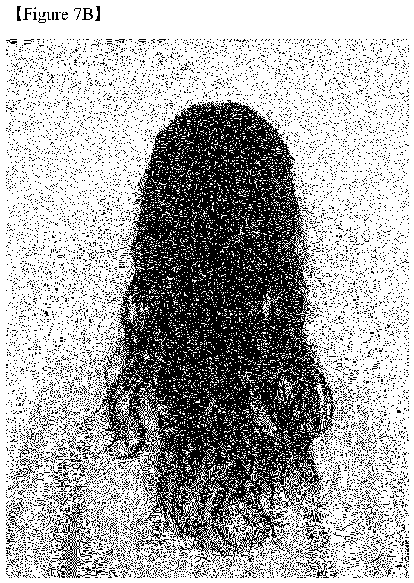

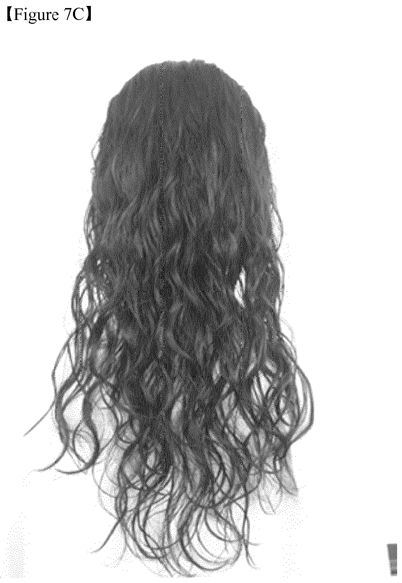

FIG. 7 is of photographs showing the results of the heat perm process by a hair expert having at least 20 years of experience in a hair salon in Gangnam-gu, Korea, using hydrogen water having a dissolved hydrogen content (L) of about 4,400 ppb (operation of a hydrogen water generator for about 150 min) as the oxidizing agent. The female resident of Seoul in her twenties was subjected to the process of FIG. 2. FIG. 7A shows a photograph of the hair before the heat perm process and FIG. 7B shows a photograph of the hair after the heat perm process. FIG. 7C is a brightly photographed image taken using a flash so that the shape of the curl is visible to the naked eye. FIG. 7 shows the hair curl that is formed before (7A) and after (7B) the heat perm process. As shown in FIG. 7, the hair curl is well formed, and thus HW can be confirmed to be directly usable in the hair salon. Compared to when using the existing oxidizing agents (hydrogen peroxide (HO), sodium bromate (Br)), when hydrogen water was used, hair dryness and loosening were low, and hair elasticity was good. Furthermore, in the opinion of the person who was subjected to the heat perm process using hydrogen water (HW) as the oxidizing agent, there was almost no scalp stinging or itching compared to existing hair perm processes, and also, there was almost no smell from the scalp or hair after the heat perm process. The hair condition was continuously measured for about 3 months after the heat perm process using hydrogen water (HW) as the oxidizing agent. As the results thereof, hair curl sustainability was improved compared to when using hydrogen peroxide (HO) and sodium bromate (Br) as oxidizing agents.

Therefore, hydrogen water (HW) as the unique oxidizing agent can alleviate the side effects of existing oxidizing agents for forming a hair perm, such as hydrogen peroxide (HO) and sodium bromate (Br), on the scalp and hair and can exhibit outstanding curl formability and sustainability.

## Claims

1. A method of forming a hair perm using an oxidizing agent, wherein the oxidizing agent consists of hydrogen water (HW), and the hydrogen water (HW) has a pH of 7.0 to 7.9.

2. The method of claim 1, wherein the hydrogen water (HW) has a dissolved hydrogen content of at least 100 ppb.

3. The method of claim 1, wherein the hydrogen water (HW) has a dissolved hydrogen content of 400 ppb to 4,400 ppb.

## Patentansprüche

1. Verfahren zur Herstellung einer Dauerwelle unter Verwendung eines Oxidationsmittels, wobei das Oxidationsmittel aus Hydrogenwasser (HW) besteht, und das Hydrogenwasser (HW) einen pH-Wert von 7,0 bis 7,9 aufweist.

2. Verfahren nach Anspruch 1, wobei das Hydrogenwasser (HW) einen Gehalt an gelöstem Wasserstoff von mindestens 100 ppb aufweist.

3. Verfahren nach Anspruch 1, wobei das Hydrogenwasser (HW) einen Gehalt an gelöstem Wasserstoff von 400 ppb bis 4.400 ppb aufweist.

## Revendications

1. Procédé de formation d'une permanente capillaire utilisant un agent oxydant, dans lequel l'agent oxydant comprend de l'eau hydrogénée (HW), l'eau hydrogénée ayant un pH compris entre 7,0 et 7,9.

2. Procédé selon la revendication 1, dans lequel l'eau hydrogénée (HW) a une teneur en hydrogène dissous d'au moins 100 ppb.

3. Procédé selon la revendication 1, dans lequel l'eau hydrogénée (HW) a une teneur en hydrogène dissous comprise entre 400 ppb et 4400 ppb.
